# EUROPEAN PATENT SPECIFICATION

(11) **EP 4 082 490 B1**
(45) Date of publication and mention of the grant of the patent: **13.08.2025**
(21) Application number: 20907496.2
(22) Date of filing: 04.09.2020
(51) Int. Cl.: A61F 2/966, A61F 2/95

(54) **STENT DELIVERY SYSTEM AND METHOD FOR MOUNTING STENT**
STENTEINBRINGUNGSSYSTEM UND VERFAHREN ZUM MONTIEREN EINES STENTS
SYSTÈME DE POSE D'ENDOPROTHÈSE ET PROCÉDÉ DE MONTAGE D'ENDOPROTHÈSE

(30) Priority: 25.12.2019 CN 201911359117
(43) Date of publication of application: 02.11.2022
(73) Proprietor: Shanghai MicroPort Endovascular MedTech (Group) Co., Ltd., Shanghai 201318 (CN)
(72) Inventor: PENG, Dadong, Shanghai 201318 (CN); TU, Chunlin, Shanghai 201318 (CN); DENG, Xiaojuan, Shanghai 201318 (CN); YUAN, Zhenyu, Shanghai 201318 (CN); ZHU, Qing, Shanghai 201318 (CN)
(74) Representative: Patentship Patentanwaltsgesellschaft mbH
(86) International application number: PCT/CN2020/113612
(87) International publication number: WO 2021/128937

(56) References cited:
- EP-A1- 2 298 248
- WO-A1-2019/232155
- CN-A- 102 014 805
- CN-A- 104 706 449
- CN-A- 105 943 211
- CN-A- 107 405 206
- CN-A- 109 350 317
- CN-A- 110 022 795
- CN-A- 111 035 486
- US-A1- 2014 180 386
- US-A1- 2019 321 207
- US-B1- 6 302 891

## Description

### TECHNICAL FIELD

The present invention relates to the field of medical devices and, in particular, to a stent delivery system and a method for mounting stent.

### BACKGROUND

Thoracic aortic aneurysms or dissections are a fatal vascular surgical disease that is rather prevalent. Before the 1990s, thoracic aortic aneurysms or dissections were treated using traditional surgical techniques, which were, however, associated with a range of disadvantages including high surgical difficulty, significant trauma and a large number of complications. Since in 1994, Dake and his colleagues first reported treating an aortic dissection with an implanted endovascular covered stent, interventional surgery has been greatly developed. Such an interventional surgical procedure typically involves introducing a covered stent into a patient's body through an outer sheath inserted via a puncture incision made in the femoral artery and advancing it through a main artery to a target lesion site in the aorta. Afterward, the covered stent is released there to cover and repair the diseased vessel section. A covered stent is a prosthetic implant in which an inner or outer surface of a stent body is partially or entirely covered with a membrane.

Treating an aortic dissection with a covered stent consists of delivering the covered stent to the target lesion site using a special delivery system, followed by release and expansion of the covered stent. As a result, the membrane in the covered stent closes the entry tear in the aorta, blocking blood from further entering the false lumen in the dissection and preventing further extension of the tear or even rupture of the aorta. At the same time, the stent body in the covered stent supports the vessel wall, thereby preventing blood from flowing into the false lumen and compressing the true lumen, which may affect normal blood flow and decrease blood supply to affected tissues and organs. Closing the entry tear typically requires a length of healthy blood vessel to be present around a proximal end of the covered stent, which can serve as a stent anchor. However, since the entry tear is often close to the orifices of important branch vessels, such an anchor tends to be very short, limited in length and precious. Additionally, due to forces from blood flow or system design issues, it is usually difficult to release the covered stent exactly to the target site, and the deployed covered stent is often more or less displaced forward or backward from the target position, possibly leading to complete closure of the stent anchor as well as the surrounding branch vessels, which may become a cause of organ ischemia, cerebral ischemia or other undesirable consequences that are detrimental to the patient's health, or to incomplete closure of the entry tear, which may become a cause of endoleak or other undesirable consequences that are detrimental to the patient's health.

US 2019/321207 A1 discloses a delivery system and method for implanting a stent graft prosthesis.

### SUMMARY OF THE INVENTION

The present invention is set out in the appended set of claims.

It is an objective of the present invention to provide a stent delivery system and a stent loading method, which allow enhanced positional accuracy of a released stent by preventing the stent from moving backward under the action of forces from blood flow.

To this end, the provided stent delivery system includes a handle, an outer tube, a restraining member and a delayed-release member.

The outer tube defines an inner lumen extending in an axial direction for receiving a stent in a crimped state, the outer tube configured to withdraw toward a distal end of the stent under a control of the handle so that the stent is exposed.

The restraining member includes at least one restraining thread and a control guidewire, the restraining thread arranged circumferentially around the stent, the control guidewire configured to switch the restraining thread between an open-loop or closed-loop structure for compressing or releasing the stent.

The delayed-release member is disposed within the outer tube and configured to removably connect to a proximal end of the stent to restrain or release the proximal end of the stent.

Optionally, the restraining member may include at least two restraining threads, the at least two restraining threads spaced apart from one another across a main part of the stent along the axial direction.

Optionally, each of the restraining threads may be a double-stranded restraining thread closed at both ends, wherein: the double-stranded restraining thread is configured such that a leading end thereof is inserted through a trailing end thereof after winding the double-stranded restraining thread at least one turn around the stent, while the control guidewire is inserted through the leading end of the double-stranded restraining thread, so that the double-stranded restraining thread is configured as a closed-loop structure, or the double-stranded restraining thread is configured to be wound at least one turn around the stent, while the control guidewire is inserted through both a leading end and a trailing end of the double-stranded restraining thread, so that the double-stranded restraining thread is configured as a closed-loop structure.

According to the invention, the stent is provided with a plurality of fixed coils, at least two of the plurality of fixed coils spaced apart from one another circumferentially around the stent, and wherein the restraining thread is configured to be inserted through the at least two of the plurality of fixed coils.

Optionally, the delivery system may further include an inner tube disposed within the outer tube, wherein a clearance for receiving the stent exists between the outer tube and the inner tube, and wherein the delayed-release member defines a channel for insertion of the inner tube therethrough.

Optionally, the delayed-release member may include a conical tip, a delayed-release fixture, a delayed-release rear base, a number of delayed-release screw rods and a delayed-release guidewire, the conical tip provided, at a distal end thereof, the same number of fixation holes for interacting with the respective delayed-release screw rods, the delayed-release fixture provided with the same number of guide holes for interacting with the respective delayed-release screw rods, each of the delayed-release screw rods having a distal end in fixed connection with the delayed-release rear base and a proximal end inserted through a respective one of the guide holes in the delayed-release fixture and into a respective one of the fixation holes in the conical tip, the delayed-release guidewire having a proximal end in fixed connection with the delayed-release rear base, the delayed-release screw rods having sections between the conical tip and the delayed-release fixture to be inserted through and restrain the proximal end of the stent.

Optionally, each of the conical tip, the delayed-release fixture and the delayed-release rear base may define a channel for insertion of the inner tube therethrough. The inner tube may be connected at one end to the conical tip and sequentially through the channels.

Optionally, a section of the inner tube located distally with respect to the delayed-release rear base may be provided with a limit block, the limit block having an outer diameter greater than a diameter of the channel in the delayed-release rear base.

Based on the same inventive concept, the present invention also provides a stent loading method according to claim 9, including:
securing a proximal end of a stent to a delayed-release member of a delivery system; crimping the stent with at least one restraining thread wound circumferentially around the stent, the at least one restraining thread configured as a closed-loop structure under a control of a control guidewire; and compressing and loading the crimped stent, together with an inner tube of the delivery system and the delayed-release member into an inner lumen of an outer tube of the delivery system, wherein a distal end of the outer tube is fixedly connected to a handle. The stent is provided with two or more fixed coils, at least two of the fixed coils are spaced apart from one another circumferentially around the stent, and the restraining thread is configured to be inserted through the at least two of the fixed coils.

Compared with the prior art, the present invention offers the following benefits:
the stent delivery system of the present invention including the handle, the outer tube, the restraining member and the delayed-release member is structurally simple and easy to use. The delayed-release member can be assembled with a proximal end portion of a stent (i.e., a portion at the end thereof closer to a target lesion site), with the restraining member binding the stent in a crimped state. This assembly can be compressed and loaded into the outer tube, delivered therein to the target lesion site and released there. During the release of the stent from the restraining member, the proximal end portion of the stent remains secured to the delayed-release member. As such, the stent will be avoided from being pushed backward by the blood, resulting in improved positional accuracy of the stent's release. Moreover, the restraining threads in the restraining member are opened and closed under the control of the control guidewire, so when the control guidewire is slowly retracted, various portions of the stent will be successively released and expand in the same pace along the direction from the proximal to distal end thereof. In this way, the stent can be released in a steady way, which ensures a further improvement in the stent's positional accuracy. In addition, a distal end of the stent can be secured to the inner tube by means of fixed coils on the stent, in addition to the securing of the stent's proximal end to the delayed-release member. This can avoid the stent from moving forward or backward during the release of the middle section thereof, allowing the stent to be released under true accurate positional control.

### BRIEF DESCRIPTION OF THE DRAWINGS

Fig. 1 schematically illustrates a stent bound by a restraining member in accordance with a specific embodiment of the present invention.
Fig. 2 schematically illustrates a stent bound by a restraining member in accordance with a specific embodiment of the present invention.
Fig. 3 schematically illustrates how a delayed-release member interacts with a proximal end portion of a stent in accordance with a specific embodiment of the present invention.
Fig. 4 is a structural schematic of a delayed-release rear base according to a specific embodiment of the present invention.
Figs. 5 to 8 are schematic illustrations of delivery of a stent using a stent delivery system according to a specific embodiment of the present invention.

In these figures,
1-stent or covered stent; 10-bare section or proximal end portion of the stent; 11-middle section of the stent; 12-distal end portion of the stent; 21-restraining thread; 211-leading end of the restraining thread; 212-trailing end of the restraining thread; 22-control guidewire; 31-fastener on the stent; 32-fixed coil; 4-delayed-release member; 41-conical tip; 410-fixation hole in the conical tip; 42-delayed-release fixture; 421-guide hole in the delayed-release fixture; 43-delayed-release screw rod; 44-delayed-release rear base; 441-channel in the delayed-release rear base; 442-first bore; 443-second bore; 45-limit block; 46-delayed-release guidewire; 51-inner tube; 52-outer tube; 6-positioning guidewire; 7-blood vessel.

### DETAILED DESCRIPTION

Objectives and features of the present invention will become more readily apparent upon reading the following detail description of a few embodiments in conjunction with the accompanying drawings. However, the present invention may be embodied in various other forms and is not limited to the disclosed embodiments. As used herein, the term "proximal end" refers to an end of a medical instrument that is located closer to where it is deployed (e.g., the heart, a diseased section of a blood vessel or the like) during normal operation thereof, which is also a leading end of a stent delivery system in the advancement direction thereof, and an end located away from a person operating the medical instrument. On the contrary, a "distal end" often refers to an end of the medical instrument that is located farther away from where it is deployed, which is also an end closer to the operator, and an end closer to a handle of the medical instrument.

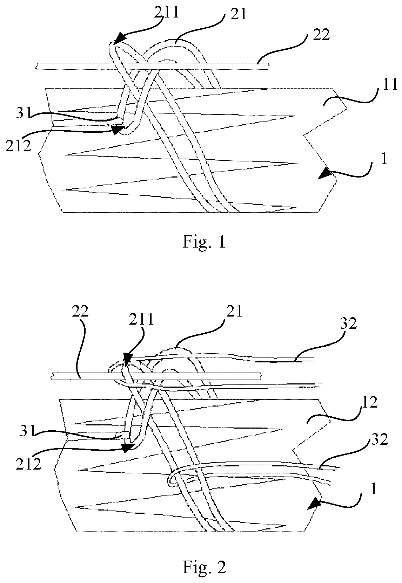

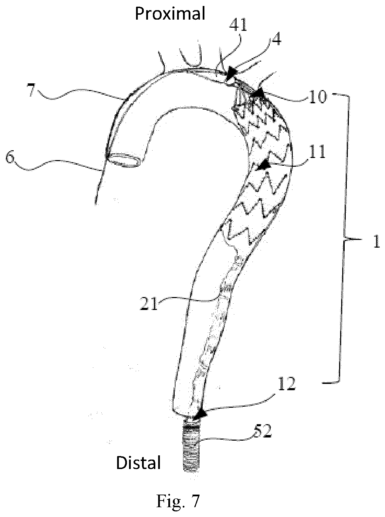

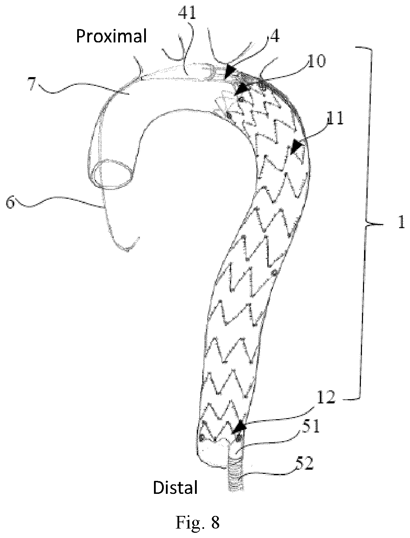

Referring to Figs. 1 to 8, in an embodiment of the present invention, there is provided a stent delivery system including a handle, an outer tube 52, a restraining member and a delayed-release member 4.

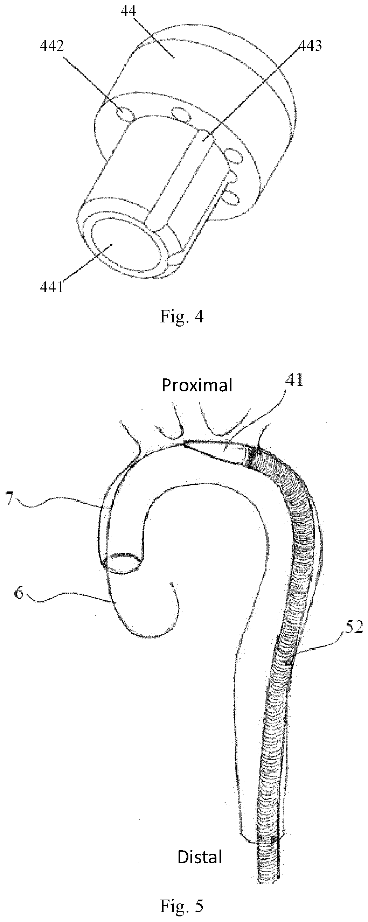

Referring to Figs. 5 to 7, the outer tube 52 defines an axial inner lumen for housing a crimped stent 1 and is configured to withdraw toward a distal end of the stent 1 when the handle is manipulated, so that the stent 1 is exposed. The stent delivery system according to this embodiment also includes an inner tube 51, which is disposed within the outer tube 52 so that there is a clearance left between the inner tube 51 and the outer tube 52, and the stent 1 can be received in the clearance. The delayed-release member 4 defines a channel allowing passage of the inner tube 51, and a proximal end of the inner tube 51 is fixedly connected to a proximal end of the delayed-release member 4. The stent 1 can be disposed over the inner tube 51, with a proximal end portion thereof being assembled with the delayed-release member 4 and the rest portion being bound by the restraining member in the crimped state. A distal end portion and a middle section of such an assembly of the stent 1, the restraining member and the delayed-release member 4 can be together movably received in the clearance between the outer tube 52 and the inner tube 51.

According to this embodiment, the stent 1 that can be delivered by the stent delivery system may be either a covered stent or an uncovered or bare stent. The bare stent is generally a hollow tubular structure made of stainless steel and/or a metallic shape memory material, which can be disposed over the inner tube 51 and is not covered by a polymer membrane. In order to deliver the bare stent into a blood vessel, it can be crimped into an unexpanded configuration that facilitates the bare stent's delivery. Once it is delivered to a target lesion site in the blood vessel, the bare stent can expand to a configuration conforming to the curved anatomy of the blood vessel, without exerting local forces on the vessel wall due to resilience, which may cause the occurrence of complications. The bare stent may be, for example, a hollow tubular structure consisting of zigzag rings interconnected alternately with metal mesh rings, or a hollow tubular structure made of a metal mesh. A covered stent differs from a bare stent in having a metal stent body covered by a membrane. The membrane can entirely cover an outer or inner surface of the metal stent body. Alternatively, it can cover only a middle section of the metal stent body. Still alternatively, it can cover both a middle section and a distal end portion of the metal stent body. Still yet alternatively, it may even cover multiple portions of a middle section of the metal stent body in a discontinued manner. The membrane may also be generally a hollow tubular structure and can be disposed over the inner tube 51. A covered stent can more easily switch between a curved, expanded configuration and a crimped, unexpanded configuration. The unexpanded configuration can facilitate delivery of the covered stent into a blood vessel. Upon reaching the target lesion site in the blood vessel, the covered stent may be released so that it switches to the expanded configuration in conformance with the curved anatomy of the blood vessel, without exerting local forces on the vessel wall due to resilience, which may cause the occurrence of complications. A covered stent usually includes a metal stent body and a membrane covering an inner or outer surface of the metal stent body. The metal stent body may be an elastic structure with openings in the wall, made of a metallic material such as stainless steel or a metallic shape memory material. It can be compressed and crimped, expand when released, telescope axially and be corrugated radially. Therefore, it can be passively adapted in diameter and/or length, making the covered stent better fit to the geometry of a blood vessel in which it is deployed and thus resulting in a reduced risk of endoleak. The metal stent body may be a metal mesh braided from metal wire(s), or a structure consisting of several zigzag rings fixedly connected together by multiple struts, or a structure consisting of zigzag rings and metal mesh rings that are connected together by multiple struts.

In the embodiment shown in Figs. 1 to 8, the stent 1 is a covered stent consisting essentially of a metal stent body and a membrane covering a surface of the metal stent body. The stent 1 has a proximal end portion 10 proximal to a target lesion site, a distal end portion 12 located away from the target lesion site and a middle section 11 between the proximal and distal end portions 10, 12. The proximal end portion of the stent 1 is a bare stent section not covered by the membrane, while an outer surface of the metal stent body of the stent 1 is covered by the membrane both at the middle section 11 and the distal end portion 12. Preferably, the metal stent body of the stent 1 is fabricated from a metallic shape memory material such as a nickel-titanium (NiTi) alloy, which has shape memory and can avoid the issue of unsatisfactory deformability arising from the use of other materials. The membrane of the stent 1 is made of a polymeric material such as polyester (PET), polytetrafluoroethylene (PTFE), nylon, terylene, polypropylene, etc. The membrane may be either sewn with polyester sutures, or press-molded, onto the metal stent body. Preferably, the membrane is provided with radiopaque markers made of, for example, a platinum iridium alloy, which enable a user to dynamically monitor the position of the stent 1 during its introduction or release by means of radiopaque equipment. This allows adjusting the stent 1 to an optimal position, making it possible to release the stent with high accuracy.

In the embodiment shown in Figs. 1 to 8, the bare stent section 10, i.e., the proximal end portion, of the stent, is configured to strengthen anchoring of the stent 1 to a vessel wall. It may include at least one wave-shaped ring that is not covered by the membrane. The wave-shaped ring may be braided from metal wire(s) or fabricated by cutting a metal tube. Alternatively, it may include multiple metal wires that are so individually orientated that they generally resemble a claw, or be made of a metal mesh. Preferably, according to the present invention, the bare stent section 10 is provided with a biocompatible barrier layer made preferably of PTFE. The PTFE layer may be formed by winding a strip of PTFE film on the surface of the metal wire(s) or tube of the bare stent section 10. Alternatively, it may also be formed by spring liquid PTFE onto the surface of the bare stent section 10. This barrier layer can prevent thrombogenesis on the surface of the bare stent section, inhibit the release of divalent nickel ions and the like and protect the bare stent section 10 from chloride and other corrosive ions in the body fluids. It has good thrombogenetic performance, corrosion resistance and ability to prevent dissolution and release of toxic metal ions. The bare stent section 10 may have a straight cylindrical shape, or a flare shape tapered from a distal end to a proximal end, or a conical shape tapered from a distal end to a proximal end. Additionally, the bare stent section 10 may be fixed at multiple points to the membrane of the stent 1, or integrated with, welded to, or otherwise coupled to the part of the metal stent body that is covered by the membrane.

Referring to Figs. 1 to 8, the restraining member is provided on the middle section 11 and the distal end portion 12 of the stent 1 and may assume an opened or closed configuration with respect to the middle section 11 and the distal end portion 12 of the stent 1. It is configured to bind, in the closed configuration, the middle section 11 and the distal end portion 12 of the stent 1 in a crimped state, and allow, in the opened configuration, the middle section 11 and the distal end portion 12 of the stent 1 to be released and expand. The restraining member includes at least one restraining thread 21 and a control guidewire 22. The restraining thread 21 is arranged to circumferentially surround the stent 1, and the control guidewire 22 is configured to configure the restraining thread 21 as an open- or closed-loop structure for compressing or releasing the stent 1.

In the embodiment shown in Figs. 1 and 2, the restraining member includes at least two restraining threads 21, all of the restraining threads 21 are spaced from one another along an axial direction of the metal stent body of the stent 1 (i.e., a main part of the stent 1) and configured to restrict a diameter of the stent 1 to a desired value under the control of the control guidewire 22. The spacing(s) between adjacent restraining threads 21 (i.e., portion(s) of the stent 1 that are not wound by the restraining threads) are provided to avoid tangling between the restraining threads 21 or between the control guidewire 22 and the restraining threads 21. Preferably, the restraining threads 21 are formed of a material that can and will be metabolized and absorbed in the human body. In this embodiment, each restraining thread 21 is double-stranded and closed at both ends, and may be configured to be wound at least one turn around the stent 1, with its leading end 211 being inserted through its trailing end 212 and with the control guidewire 22 being, in turn, inserted through the leading end 211. In this way, it becomes a closed-loop structure. Alternatively, each double-stranded restraining thread may be configured to be wound at least one turn around the stent, with the control guidewire 22 being inserted through both its leading and trailing ends 211, 212 so that it also becomes a closed-loop structure. In addition, the individual restraining threads 21 may define equal or unequal diameters when they are wound on the stent 1. In this embodiment, when the control guidewire 22 is retracted toward the distal end of the stent 1, the restraining threads 21 will successively become open-loop structures in the direction from the proximal to the distal end of the stent. As a result, the stent 1 will expand and be thus released.

In alternative embodiments of the present invention, each restraining thread 21 may be single-stranded and configured to be wound around a desired portion of the stent 1 at least one turn so as to bind the stent 1 in a crimped state. Additionally, trailing and leading ends of each restraining thread 21 may be tied on the control guidewire 22 so that it can become, under the control of the control guidewire 22, an open- or closed-loop structure for compressing or releasing the stent 1.

Preferably, the stent 1 may be provided with fasteners 31 such as anchoring hooks or securing eyelets projecting from the metal stent body of the stent 1. When the trailing ends of the restraining threads 21 are inserted through the fasteners 31 on the stent 1, the open- or closed-loop structures formed by the restraining threads 21 will not stray away. This, on the one hand, can facilitate the control of the control guidewire 22 over the leading and trailing ends of the restraining threads 21 and, on the other hand, can avoid tangling between the restraining threads 21 and between the control guidewire 22 and the restraining threads 21.

Referring to Fig. 2, in this embodiment, at least one fixed coil 32 may be provided on the distal end portion 12 of the stent 1. Each restraining thread 21 is configured to be inserted through a corresponding one of the fixed coil(s) 32. Each fixed coil may have a distal end fixed to a distal end of the inner tube 51. In the case of two or more such fixed coils being provided, the fixed coils 32 are preferably distributed evenly around a circumference of the distal end portion 12 of the stent 1. In this way, the distal end portion 12 of the stent 1 will be uniformly stressed, avoiding the stent 1 from rotating or otherwise moving due to unbalanced stressing. In addition, the control guidewire 22 may be inserted through proximal ends of some of the fixed coils 32, with the restraining threads 21 being each inserted through a proximal end of a corresponding one of the remaining fixed coils 32. In this case, when the control guidewire 22 is withdrawn, the fixed coils 32 with their proximal ends through which the control guidewire 22 was inserted will be freed, and the fixed coils 32 with their proximal ends through which the respective restraining threads 21 were inserted will be pushed aside due to the elastic expansion of the stent 1. As a result, the stent 1 is released at the distal end portion 12 from the restraining threads 21. At the same time, since the distal ends of the fixed coils 32 with their proximal ends through which the respective restraining threads 21 were inserted are still being fixed to the inner tube 51, it is ensured that the distal end portion 12 of the stent 1 stays at the same position as before it was released, without retracting or otherwise moving during the release and elastic expansion of the stent 1.

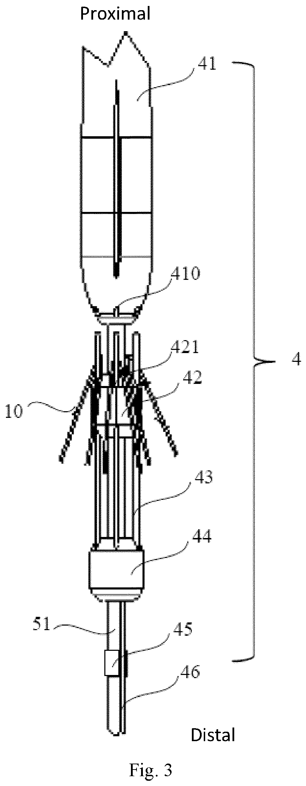

In the embodiment shown in Figs. 3 and 4, the delayed-release member 4 is configured to restrain and release the proximal end portion (i.e., the bare stent section) of the stent 1, and the delayed-release member 4 has components that are respectively moveably disposed over the inner tube 51 and fixed to the inner tube 51. In particular, the delayed-release member 4 may include a conical tip 41, a delayed-release fixture 42, a delayed-release rear base 44, a number of delayed-release screw rods 43 and delayed-release guidewire(s) 46. The conical tip 41 is fixedly disposed at the proximal end of the inner tube 51 and has a proximal end (i.e., the diametrically smaller end), which acts as a leading end of the delivery system for guiding the advancement thereof. This end can facilitate delivery of the stent 1 loaded in the delivery system by reducing resistance in a blood vessel in which the system is advancing. The same number of fixation holes 410 as the delayed-release screw rods 43 are provided at a distal end of the conical tip 41 (i.e., the diametrically large end that is closer to the operator), and the same number of guide holes 421 as the delayed-release screw rods 43 are provided in the delayed-release fixture 42. Each of the delayed-release screw rods 43 has a distal end fixed to the delayed-release rear base 44 and a proximal end that is inserted through a respective one of the guide holes 421 in the delayed-release fixture 42 and received in a respective one of the fixation holes 410 in the conical tip 41. A proximal end of each delayed-release guidewire 46 is fixed to the delayed-release rear base 44. The bare stent section 10 at the proximal end of the covered stent 1 is configured to be disposed over and crimped on sections of the delayed-release screw rods 43 between the conical tip 41 and the delayed-release fixture 42. In alternative embodiments, the guide holes 421 in the delayed-release fixture 42 may be replaced with other structures such as ridges or slide channels.

In order to load the covered stent 1, the bare stent section 10 at its proximal end is disposed over the delayed-release rods 43, and the delayed-release rear base 44 is then pushed toward the conical tip 41 until a limit is reached. Subsequently, the delayed-release screw rods 43 are inserted into the respective fixation holes 410 in the conical tip 41 under the guide of the respective guide holes 421 in the delayed-release fixture 42. In this way, the bare stent section 10 is secured on the delayed-release screw rods 43 between the conical tip 41 and the delayed-release fixture 42. In order to release the covered stent 1, the handle is manipulated to retract the delayed-release guidewire(s) 46 so that the delayed-release screw rods 43 are removed from the fixation holes 410 in the conical tip 41, allowing release of the bare stent section 10 at the proximal end of the covered stent 1. As a result, the bare stent section expands by means of its own resilience and firmly adheres to the wall of the blood vessel, thus securing the covered stent 1 in place. Each delayed-release guidewire 46 is fixed to a distal end of the delayed-release rear base 44.

Therefore, according to this embodiment, the delayed-release member 4 is able to restrain and release the proximal end portion of the stent 1 with the aid of the delayed-release screw rods 43, and to effectively restrain the proximal end portion of the stent 1 with the aid of the delayed-release fixture 42, with increased reliability in axial movement of the delayed-release screw rods 43. Moreover, due to minor contact areas between the delayed-release screw rods 43 and the fixation holes 410, reduced resistance will be encountered during the release of the stent 1, which results in increased accuracy of the release process.

In this embodiment, each of the conical tip 41, the delayed-release fixture 42 and the delayed-release rear base 44 defines a channel in which the inner tube 51 is moveably inserted. The inner tube 51 is inserted successively through the channels, with the inner tube 51's proximal end coupled to the distal end of the conical tip 41. The inner tube 51 is provided with a limit block 45 on a section thereof located distally with respect to the delayed-release rear base 44. The limit block 45 has an outer diameter greater than a diameter of the channel 441 in the delayed-release rear base 44. In this way, it can thus limit displacement of the delayed-release rear base 44 during the release of the bare stent section 10 (i.e., defining the aforementioned limit for the movement of the distal end of the delayed-release rear base 44) within a range that will not bring damage to surrounding blood vessels. Additionally, when the delayed-release rear base 44 is moving toward the proximal end of the stent 1 (i.e., away from the conical tip 41), the limit block 45 also provides a limit where the delayed-release rear base 44 has to stop. This prevents dislodgement of the delayed-release screw rods 43 from the delayed-release fixture 42. In this embodiment, the delayed-release screw rods 43 may be made of a metallic material such as stainless steel or a nickel-titanium alloy. In addition to meeting the requirements for loading and release of the stent, the axial length of the delayed-release screw rods 43 between the delayed-release fixture 42 and the conical tip 41 is required to be sufficiently short in order to impart increased rigidity to these sections of the delayed-release screw rods 43, which can lead to a reduced amount of deformation of the delayed-release screw rods 43 resulting from the expansion of the covered stent 1.

Further, as shown in Fig. 4, the channel 441 is formed at a center of the delayed-release rear base 44, and a number of first bores 442 for fixing the delayed-release rods 43 are uniformly distributed around the channel 441. The number of the first bores 442 may be six, for example. The first bores 442 are equiangularly distributed circumferentially around the channel 441, and the number of the first bores 442 is the same as that of the delayed-release screw rods 43. The delayed-release screw rods 43 are fixed in the respective first bores 442. At least one second bore 443 for fixing the delayed-release guidewire(s) 46 is also formed circumferentially around the channel 441 in the delayed-release rear base 44. The number of the second bore(s) 443 is the same as that of the delayed-release guidewire(s) 46, each delayed-release guidewire 46 is inserted into one second bore 443 from the distal to proximal end of the delayed-release rear base 44 and is then bent over into another second bore 443. The delayed-release guidewire(s) 46 may be secured in the second bore(s) 443 by any of methods including, but not limited to, welding, bonding with an adhesive, mechanical locking.

Referring to Figs. 1 to 8, in an embodiment of the present invention, there is also provided a method for loading, delivery and release of the stent. With the stent 1 being implemented as a covered stent (referred hereinafter to as the "covered stent 1") as an example, the method may include the steps as detailed below.

The loading of the covered stent 1 may be accomplished by the following three steps. In step 1), the proximal end portion of the covered stent 1 is retained on the delayed-release member. Specifically, the delayed-release member 4 is assembled with the inner tube 51 in advance, with the delayed-release screw rods 43 being inserted through the respective guide holes 421 in the delayed-release fixture 42, the covered stent 1 is then disposed over the inner tube 51 and moved on the inner tube 51 until the covered stent 1's proximal end reaches a location near the proximal end of the delayed-release member 4. The proximal end portion (i.e., the bare stent section) 10 of the covered stent 1 is then sleeved over the delayed-release screw rods 43, and the delayed-release guidewire(s) 46 of the delayed-release member 4 is/are then actuated so that the delayed-release rear base 44 of the delayed-release member 4 moves over the inner tube 51 toward the conical tip 41. Driven by the delayed-release rear base 44 and guided by the guide holes 421 in the delayed-release fixture 42, the delayed-release screw rods 43 are inserted into the respective fixation holes 410 in the conical tip 41, thus securing the bare stent section 10 of the covered stent 1. In step 2), the covered stent 1 is bound with the restraining threads 21. Specifically, the restraining threads 21 are circumferentially arranged on the stent by suturing and winding, and configured as multiple closed loop structures (i.e., thread rings) under the control of the control guidewire 22. These closed loop structures can confine the middle section 11 and the distal end portion 12 of the stent 1 over the inner tube 51 in a crimped state. For example, each restraining thread 21 may be double-stranded, closed at both ends and configured as a closed-loop structure by winding the restraining thread 21 at least one turn around the stent, with its leading end 211 being inserted through its trailing end 212 and with the control guidewire 22 being, in turn, inserted through the leading end 211. As such, the proximal end portion of the covered stent 1 is secured to the delayed-release member 4, and the middle section 11 and the distal end portion 12 of the covered stent 1 are fixed by the restraining threads and the control guidewire 22. In step 3), the covered stent 1 is compressed and loaded into the outer tube 52. Specifically, the assembly of the delayed-release member 4, the restraining member, the covered stent 1 and the inner tube 51 is compressed to a minimum possible diameter that allows it to be loaded into the inner lumen of the outer tube 52. Subsequent to the loading, the outer tube 52 is moved over the inner tube 51 until the outer tube 52's proximal end comes into contact with the proximal end of the conical tip 41, so that the covered stent 1 is entirely housed in the outer tube 52. Afterward, the distal end of the outer tube 52 is fixed to the handle.

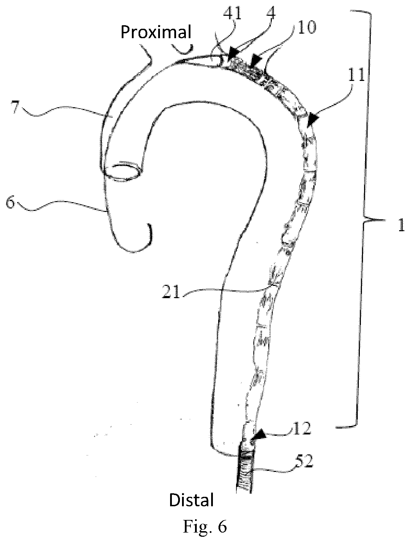

The delivery and release of the covered stent 1 may also be accomplished by three steps that correspond to the above-discussed respective steps. In step 1), the covered stent 1 is delivered in the outer tube 52 to a location near the target lesion site (or designated location) in the blood vessel 7. In this way, initial positioning is accomplished, followed by withdrawal of the outer tube 52. Specifically, prior to the delivery, a leading end of a positioning guidewire 6 is introduced through a puncture incision and advanced to a location near the target lesion site in the blood vessel 7 (or designated location, e.g., an aortic dissection). During the delivery, the inner and outer tubes 51, 52 that are disposed one another and load the stent 1 in the clearance therebetween are delivered along the positioning guidewire 6 into the blood vessel 7 until the proximal end of the outer tube 52 reaches the location near the target lesion site in the blood vessel 7. In this process, since both the proximal end portion 10 (i.e., the bare stent section) and the distal end portion 12 of the covered stent 1 are secured, the covered stent 1 will not move relative to the outer tube 52 or the inner tube 51. Initial positioning is accomplished upon the covered stent 1 being delivered by the outer tube 52 to the location near the target lesion site in the blood vessel 7, as shown in Fig. 5. After that, the outer tube 52 is withdrawn, while the inner tube 51 remains retained, so that at least all the components of the covered stent 1 from the proximal end portion (i.e., the bare stent section) 10 to the distal end portion 12 are entirely exposed. Optionally, a portion of the inner tube 51 proximal to the distal end portion 12 of the covered stent 1 may be exposed, as shown in Fig. 6. At this time, the covered stent 1 is still being held in position on the inner tube 51 by the delayed-release member 4, the restraining member and the control guidewire 22. In step 2), once the covered stent 1 has been accurately positioned, the restraining threads 21 are successively loosened along the direction from the proximal to distal end of the stent 1, so that the covered stent 1 progressively expands in this direction. Specifically, the accurate positioning of the covered stent 1 is achieved by adjusting the position of the inner tube 51. Afterward, the inner tube 51 is retained, concurrently with the control guidewire 22 being retracted, so that the individual restraining threads 21 on the covered stent 1 are successively loosened in the direction from the proximal end portion (i.e., the bare stent section) 10 of the covered stent 1 to the distal end portion 12 of the covered stent 1. As a result, the middle section 11 and the distal end portion 12 of the covered stent 1 are consecutively released and expand to an appropriate size depending on the anatomy of the blood vessel. That is, the stent in the expanded configuration has a size adapted to that of the blood vessel 7. The speed of release of the covered stent 1 depends on how fast the control guidewire 22 is withdrawn. The faster the control guidewire 22 is withdrawn, the speedier the release of the covered stent 1 will be. In step 3), delayed release of the proximal end portion (i.e., the bare stent section) 10 of the covered stent 1 is accomplished by the delayed-release member 4. Specifically, the delayed-release guidewire(s) 46 in the delayed-release member 4 is/are actuated to drive the delayed-release rear base 44 away from the conical tip 41, causing the delayed-release screw rods 43 to dislodge from the fixation holes 410 in the conical tip 41 and dock back into the guide holes 421 in the delayed-release fixture 42. As a result, the proximal end portion (i.e., the bare stent section) 10 of the covered stent 1 is totally released from the delayed-release screw rods 43. In this way, the proximal end portion (i.e., the bare stent section) 10 of the covered stent 1 is completely removed from the delayed-release member 4. The released proximal end portion (i.e., the bare stent section) 10 of the covered stent 1 will expand and anchor to the tissue at the target lesion site in the blood vessel 7, thereby securing the covered stent 1 in the blood vessel 7. Subsequently, the inner tube 51 is retracted until both the inner tube 51 and the delayed-release member 4 are withdrawn from the patient's body, with the restraining threads 21 remaining in the patient's body, followed withdrawal of the positioning guidewire 6. In this way, the covered stent 1 is implanted in the patient's body.

In summary, the stent delivery system of the present invention including the handle, the outer tube, the restraining member and the delayed-release member is structurally simple and easy to use. The delayed-release member can be assembled with a proximal end portion of a stent (i.e., a portion at the end thereof closer to a target lesion site), with the restraining member binding the stent in a crimped state. This assembly can be compressed and loaded into the outer tube, delivered therein to the target lesion site and released there. During the release of the stent's middle section from the restraining member, the proximal end portion of the stent remains secured to the delayed-release member. As such, the stent will be avoided from being pushed backward by the blood, resulting in improved positional accuracy of the stent's release. Moreover, the restraining threads in the restraining member are opened and closed under the control of the control guidewire, so when the control guidewire is slowly retracted, various portions of the stent will be successively released and expand in the same pace along the direction from the proximal to distal end thereof. In this way, the stent can be released in a steady way, which ensures a further improvement in the stent's positional accuracy. In addition, a distal end of the stent can be secured to the inner tube by means of fixed coils on the stent, in addition to the securing of the stent's proximal end to the delayed-release member. This can avoid the stent from moving forward or backward during the release of the middle section thereof, allowing the stent to be released under true accurate positional control.

Apparently, those skilled in the art can make various modifications and variations to the present invention without departing from the scope thereof. Accordingly, the invention is intended to embrace all such modifications and variations if they fall within the scope of the appended claims.

## Claims

1. A stent delivery system, comprising a handle, an outer tube (52), a restraining member and a delayed-release member (4),
the outer tube (52) defining an inner lumen extending in an axial direction for receiving a stent (1) in a crimped state, the outer tube (52) configured to withdraw toward a distal end of the stent (1) under a control of the handle so that the stent (1) is exposed;
the restraining member comprising at least one restraining thread (21) and a control guidewire (22), the restraining thread (21) arranged circumferentially around the stent (1), the control guidewire (22) configured to switch the restraining thread (21) between an open-loop or closed-loop structure for compressing or releasing the stent (1);
the delayed-release member (4) disposed within the outer tube (52) and configured to removably connect to a proximal end of the stent (1) to restrain or release the proximal end of the stent (1), **characterized in that**:
the stent (1) is provided with two or more fixed coils (32), at least two of the fixed coils (32) are spaced apart from one another circumferentially around the stent (1), and the restraining thread (21) is configured to be inserted through the at least two of the fixed coils (32).

2. The stent delivery system of claim 1, wherein the restraining member comprises at least two restraining threads (21), the at least two restraining threads (21) spaced apart from one another across a main part of the stent (1) along the axial direction.

3. The stent delivery system of claim 1, wherein each of the restraining threads is a double-stranded restraining thread closed at both ends, wherein:
the double-stranded restraining thread is configured such that a leading end thereof is inserted through a trailing end thereof after winding the double-stranded restraining thread at least one turn around the stent (1), while the control guidewire (22) is inserted through the leading end of the double-stranded restraining thread, so that the double-stranded restraining thread is configured as a closed-loop structure.

4. The stent delivery system of claim 1, wherein each of the restraining threads is a double-stranded restraining thread closed at both ends, wherein the double-stranded restraining thread is configured to be wound at least one turn around the stent (1), while the control guidewire (22) is inserted through both a leading end and a trailing end of the double-stranded restraining thread, so that the double-stranded restraining thread is configured as a closed-loop structure.

5. The stent delivery system of claim 1, further comprising an inner tube (51) disposed within the outer tube (52), wherein a clearance for receiving the stent (1) exists between the outer tube (52) and the inner tube (51), and wherein the delayed-release member (4) defines a channel (441) for insertion of the inner tube (51) therethrough.

6. The stent delivery system of claim 5, wherein the delayed-release member (4) comprises a conical tip (41), a delayed-release fixture, a delayed-release rear base (44), a number of delayed-release screw rods (43) and a delayed-release guidewire (46), the conical tip (41) provided, at a distal end thereof, the same number of fixation holes (410) for interacting with the respective delayed-release screw rods (43), the delayed-release fixture provided with the same number of guide holes (421) for interacting with the respective delayed-release screw rods (43), each of the delayed-release screw rods (43) having a distal end in fixed connection with the delayed-release rear base (44) and a proximal end inserted through a respective one of the guide holes (421) in the delayed-release fixture and into a respective one of the fixation holes (410) in the conical tip (41), the delayed-release guidewire (46) having a proximal end in fixed connection with the delayed-release rear base (44), the delayed-release screw rods (43) having sections between the conical tip (41) and the delayed-release fixture to be inserted through and restrain the proximal end of the stent (1).

7. The stent delivery system of claim 6, wherein each of the conical tip (41), the delayed-release fixture and the delayed-release rear base (44) defines a channel (441) for insertion of the inner tube (51) therethrough, and wherein the inner tube (51) is connected at one end to the conical tip (41) and sequentially through the channels (441).

8. The stent delivery system of claim 7, wherein a section of the inner tube (51) located distally with respect to the delayed-release rear base (44) is provided with a limit block (45), the limit block (45) having an outer diameter greater than a diameter of the channel (441) in the delayed-release rear base (44).

9. A method for mounting a stent (1), comprising:
securing a proximal end of a stent (1) to a delayed-release member (4) of a delivery system;
crimping the stent (1) with at least one restraining thread (21) wound circumferentially around the stent (1), the at least one restraining thread (21) configured as a closed-loop structure under a control of a control guidewire (22); and
compressing and loading the crimped stent (1), together with an inner tube (51) of the delivery system and the delayed-release member (4) into an inner lumen of an outer tube (52) of the delivery system, wherein a distal end of the outer tube (52) is fixedly connected to a handle, **characterized in that**:
the stent (1) is provided with two or more fixed coils (32), at least two of the fixed coils (32) are spaced apart from one another circumferentially around the stent (1), and the restraining thread (21) is configured to be inserted through the at least two of the fixed coils (32).

## Patentansprüche

1. Ein Stenteinbringungssystem, das einen Griff, ein Außenrohr (52), ein Rückhalteelement und ein verzögert freisetzendes Element (4) umfasst,
wobei das Außenrohr (52) ein inneres Lumen definiert, das sich in axialer Richtung zur Aufnahme eines Stents (1) in einem gecrimpten Zustand erstreckt, wobei das Außenrohr (52) ausgebildet ist, dass es sich unter Kontrolle des Griffs zum distalen Ende des Stents (1) zurückzieht, so dass der Stent (1) ausgesetzt ist;
wobei das Rückhalteelement mindestens einen Rückhaltefaden (21) und einen Steuerführungsdraht (22) umfasst, wobei der Rückhaltefaden (21) kreisförmig um den Stent (1) angeordnet ist, wobei der Steuerführungsdraht (22) ausgebildet ist, den Rückhaltefaden (21) zwischen einer offenen oder geschlossenen Schleifenstruktur zum Komprimieren oder Freigeben des Stents (1) umzuschalten;
wobei das verzögert freisetzende Element (4) innerhalb des Außenrohrs (52) angeordnet ist und so ausgebildet ist, abnehmbar mit einem proximalen Ende des Stents (1) zu verbinden, um das proximale Ende des Stents (1) festzuhalten oder freizugeben (1), **dadurch gekennzeichnet, dass**:
der Stent (1) mit zwei oder mehr festen Spulen (32) ausgestattet ist, mindestens zwei der festen Spulen (32) im Umfang des Stents (1) voneinander beabstandet sind, und der Rückhaltefaden (21) so konfiguriert ist, dass er durch die mindestens zwei der festen Spulen eingeführt wird (32).

2. Das Stenteinbringungssystem nach Anspruch 1, wobei das Rückhalteelement mindestens zwei Rückhaltefäden (21) umfasst, wobei die mindestens zwei Rückhaltefäden (21) über einen Hauptteil des Stents (1) hinweg entlang der axialen Richtung voneinander beabstandet sind.

3. Das Stenteinbringungssystem nach Anspruch 1, wobei jeder der Rückhaltefäden ein doppelsträngiger Rückhaltefaden ist, der an beiden Enden geschlossen ist, wobei:
der doppelsträngige Rückhaltefaden so konfiguriert ist, dass, nach dem Aufwickeln des doppelsträngigen Rückhaltefadens um mindestens eine Windung um den Stent (1), ein vorderes Ende davon durch ein hinteres Ende davon eingeführt wird, während der Steuerführungsdraht (22) durch das vordere Ende des doppelsträngigen Rückhaltefadens eingeführt wird, so dass der doppelsträngige Rückhaltefaden als geschlossene Schleifenstruktur ausgebildet ist.

4. Das Stenteinbringungssystem nach Anspruch 1, wobei jeder der Rückhaltefäden ein doppelsträngiger Rückhaltefaden ist, der an beiden Enden geschlossen ist, wobei der doppelsträngige Rückhaltefaden so konfiguriert ist, dass er mindestens eine Umdrehung um den Stent (1) gewickelt wird, während der Steuerführungsdraht (22) sowohl durch ein vorderes Ende als auch durch ein hinteres Ende des doppelsträngigen Rückhaltefadens eingeführt wird, so dass der doppelsträngige Rückhaltefaden als geschlossene Schleifenstruktur ausgebildet ist.

5. Das Stenteinbringungssystem nach Anspruch 1, das weiterhin ein Innenrohr (51) umfasst, das innerhalb des Außenrohrs (52) angeordnet ist, wobei ein Freiraum zur Aufnahme des Stents (1) zwischen dem Außenrohr (52) und dem Innenrohr (51) besteht, und wobei das verzögert freisetzende Element (4) einen Kanal (441) zum Einführen des Innenrohrs hindurch definiert (51).

6. Das Stenteinbringungssystem nach Anspruch 5, wobei das verzögert freisetzende Element (4) eine konische Spitze (41), eine Vorrichtung zur verzögerten Freisetzung, eine hintere Basis (44) zur verzögerten Freisetzung, mehrere Schraubenstangen (43) zur verzögerten Freisetzung und einen Führungsdraht (46) zur verzögerten Freisetzung umfasst, wobei: die konische Spitze (41) an ihrem distalen Ende mit der gleichen Anzahl von Befestigungslöchern (410) zur Interaktion mit den jeweiligen Schraubenstangen (43) zur verzögerten Freisetzung versehen wird, die Vorrichtung zur verzögerten Freisetzung mit der gleichen Anzahl von Führungslöchern (421) zur Interaktion mit den jeweiligen Schraubenstangen (43) zur verzögerten Freisetzung versehen wird, jede der Schraubenstangen (43) ein distales Ende, das fest mit der hinteren Basis (44) zur verzögerten Freisetzung verbunden ist, und ein proximales Ende umfasst, das durch ein jeweiliges Führungsloch (421) in der Vorrichtung zur verzögerten Freisetzung und in ein jeweiliges Befestigungsloch (410) in der konischen Spitze (41) eingeführt ist, der Führungsdraht (46) zur verzögerten Freisetzung ein proximales Ende aufweist, das in fester Verbindung mit der hinteren Basis (44) zur verzögerten Freisetzung ist, die Schraubenstangen (43) zur verzögerten Freisetzung Abschnitte zwischen der konischen Spitze (41) und der Vorrichtung zur verzögerten Freisetzung aufweisen, um dadurch eingeführt zu werden und das proximale Ende des Stents (1) fest zu halten.

7. Das Stenteinbringungssystem nach Anspruch 6, wobei die konische Spitze (41), die Vorrichtung zur verzögerten Freisetzung und die hintere Basis (44) zur verzögerten Freisetzung jeweils einen Kanal (441) zum Einführen des Innenrohrs (51) hindurch definieren, und wobei das Innenrohr (51) an einem Ende mit der konischen Spitze (41) und anschließend durch die Kanäle (441) verbunden ist.

8. Das Stenteinbringungssystem nach Anspruch 7, wobei ein Abschnitt des Innenrohrs (51), der distal zu der hinteren Basis (44) zur verzögerten Freisetzung angeordnet ist, mit einem Begrenzungsblock (45) versehen ist, wobei der Begrenzungsblock (45) einen Außendurchmesser aufweist, der größer als der Durchmesser des Kanals (441) in der hinteren Basis (44) zur verzögerten Freisetzung ist.

9. Ein Verfahren zum Montieren eines Stents (1) umfassend:
Sicherung eines proximalen Endes eines Stents (1) an ein verzögert freisetzendes Element (4) eines Einbringungssystems;
Crimpen des Stents (1) mit mindestens einem um den Stent (1) gewickelten Rückhaltefaden (21), wobei der mindestens eine Rückhaltefaden (21) als geschlossene Schleifenstruktur unter der Kontrolle eines Steuerführungsdrahts (22) konfiguriert ist; und
Komprimieren und Laden des gecrimpten Stents (1) zusammen mit einem Innenrohr (51) des Einbringungssystems und dem verzögert freisetzenden Element (4) in ein inneres Lumen eines Außenrohrs (52) des Einbringungssystems, wobei ein distales Ende des Außenrohrs (52) fest mit einem Griff verbunden ist, **dadurch gekennzeichnet, dass**:
der Stent (1) ist mit zwei oder mehr festen Spulen ausgestattet (32), mindestens zwei der festen Spulen (32) im Umfang des Stents (1) voneinander beabstandet sind und der Rückhaltefaden (21) so konfiguriert ist, dass er durch die mindestens zwei der festen Spulen eingeführt wird (32).

## Revendications

1. Système de pose de stent, comprenant une poignée, un tube extérieur (52), un élément de retenue et un élément à libération retardée (4),
le tube extérieur (52) définissant une lumière intérieure s'étendant dans une direction axiale pour recevoir un stent (1) dans un état serti, le tube extérieur (52) configuré pour se retirer vers une extrémité distale du stent (1) sous le contrôle de la poignée afin que le stent (1) est exposé;
l'élément de retenue comprenant au moins un fil de retenue (21) et un fil-guide de contrôle (22), le fil de retenue (21) étant disposé circonférentiellement autour du stent (1), le fil-guide de commande (22) configuré pour commuter le fil de retenue (21) entre une structure en boucle ouverte ou en boucle fermée pour comprimer ou libérer le stent (1);
l'élément à libération retardée (4) disposé à l'intérieur du tube extérieur (52) et configuré pour se connecter de manière amovible à une extrémité proximale du stent (1) pour retenir ou libérer l' extrémité proximale du stent (1), **caractérisé en ce que** :
le stent (1) est pourvu de deux ou plusieurs bobines fixes (32), au moins deux des bobines fixes (32) sont espacés les uns des autres circonférentiellement autour du stent (1), et le fil de retenue (21) est configuré pour être inséré à travers au moins deux des bobines fixes (32).

2. Système de pose de stent selon la revendication 1, dans lequel l'élément de retenue comprend au moins deux fils de retenue (21), espacés l'un de l'autre sur la partie principale du stent. (1) le long de la direction axiale.

3. Système de pose de stent selon la revendication 1, dans lequel chacun des fils de retenue est un fil de retenue à double brin fermé aux deux extrémités, dans lequel :
le fil de retenue à double brin est configuré de telle sorte qu'une extrémité avant de celui-ci est insérée à travers une extrémité arrière de celui-ci après avoir enroulé le fil de retenue à double brin au moins un tour autour du stent (1), tandis que le fil-guide de contrôle (22) est inséré à travers l' extrémité avant du fil de retenue à double brin, de sorte que le fil de retenue à double brin est configuré comme une structure en boucle fermée.

4. Système de pose de stent selon la revendication 1, dans lequel chaque fil de retenue est un fil de retenue double brin fermé à ses deux extrémités, ledit fil de retenue étant conçu pour être enroulé au moins un tour autour du stent (1), tandis que le fil-guide de contrôle (22) est inséré à la fois à travers une extrémité avant et une extrémité arrière du fil de retenue à double brin, de sorte que le fil de retenue à double brin est configuré comme une structure en boucle fermée.

5. Système de pose de stent selon la revendication 1, comprenant en outre un tube intérieur (51) disposé à l'intérieur du tube extérieur (52), dans lequel un espace libre pour recevoir le stent (1) existe entre le tube extérieur (52) et la chambre à air (51), et dans lequel l'élément à libération retardée (4) définit un canal (441) pour l'insertion du tube intérieur (51) à travers celui-ci.

6. Système de pose de stent selon la revendication 5, dans lequel l'élément à libération retardée (4) comprend une pointe conique (41), un dispositif de fixation à libération retardée, une base arrière à libération retardée (44), plusieurs tiges filetées à libération retardée (43) et un fil-guide à libération retardée (46), la pointe conique (41) comporte, à son extrémité distale, le même nombre de trous de fixation (410) destinés à interagir avec les tiges filetées à libération retardée (43), le dispositif de fixation à libération retardée comporte le même nombre de trous de guidage (421) destinés à interagir avec les tiges filetées à libération retardée (43), chacune des tiges filetées à libération retardée (43) possède une extrémité distale en liaison fixe avec la base arrière à libération retardée (44) et une extrémité proximale insérée dans l'un des trous de guidage (421) du dispositif de fixation à libération retardée et dans l'un des trous de fixation (410) de la pointe conique (41), le fil-guide à libération retardée (46) possède une extrémité proximale en liaison fixe avec la base arrière à libération retardée (44), les tiges filetées à libération retardée (43) comportant des sections entre la pointe conique (41) et le dispositif de fixation à libération retardée à insérer à travers et retenir l' extrémité proximale du stent (1).

7. Système de pose de stent selon la revendication 6, dans lequel la pointe conique (41), le dispositif de fixation à libération retardée et la base arrière à libération retardée (44) définissent chacun un canal (441) pour l'insertion du tube interne. (51) à travers celui-ci, et dans lequel le tube intérieur (51) est relié à une extrémité à la pointe conique (41) et séquentiellement à travers les canaux (441).

8. Système de pose de stent selon la revendication 7, dans lequel une section du tube intérieur (51) situé distalement par rapport à la base arrière à libération retardée (44) est pourvu d'un bloc de limite (45), le bloc de limite (45) ayant un diamètre extérieur supérieur à un diamètre du canal (441) dans la base arrière à libération retardée (44).

9. Procédé de montage d'un stent (1), comprenant :
fixation d'une extrémité proximale d'un stent (1) à un élément à libération retardée (4) d'un système d'administration ;
sertissage du stent (1) avec au moins un fil de retenue (21) enroulé circonférentiellement autour du stent (1), le ou les fils de retenue (21) étant configurés comme une structure en boucle fermée sous le contrôle d'un fil-guide de commande (22) ; et
compression et chargement du stent serti (1), avec une chambre à air (51) du système d'administration et l'élément à libération retardée (4) dans une lumière interne d'un tube externe (52) du système d'administration, dans lequel une extrémité distale de le tube extérieur (52) est relié de manière fixe à une poignée, **caractérisé en ce que** :
le stent (1) est pourvu de deux ou plusieurs bobines fixes (32), au moins deux des bobines fixes (32) sont espacés les uns des autres circonférentiellement autour du stent (1), et le fil de retenue (21) est configuré pour être inséré à travers au moins deux des bobines fixes (32).
